# EUROPEAN PATENT SPECIFICATION

(11) **EP 3 609 487 B1**
(45) Date of publication and mention of the grant of the patent: **18.03.2026**
(21) Application number: 18714289.8
(22) Date of filing: 05.04.2018
(51) Int. Cl.: A61K 31/198, A61K 31/202, A61K 31/714, A61P 25/28, A61K 31/14

(54) **OMEGA 3 FATTY ACIDS, NITRIC OXIDE RELEASING COMPOUNDS, VITAMINE B12 ET CHOLINE AS NEUROPROTECTANTS IN INDIVIDUALS WHO DO NOT HAVE DEMENTIA**
OMEGA 3 FETTENSAURE, NO FREILASSENDE VERBINDUNGEN, VITAMIN B12 UND CHOLIN ALS NEUROSCHUTZHILFSMITTEL IN PATIENTEN OHNE DEMENTIA
ACIDES GRAS OMEGA 3, COMPOSES LIBERANT NO, VITAMINE B12 ET CHOLINE COMME NEUROPROTECTEURS POUR PATIENTS SANS DEMENCE

(30) Priority: 11.04.2017 US 201762484119 P; 11.04.2017 US 201762484156 P; 29.09.2017 EP 17193916; 29.09.2017 WO PCT/EP2017/074731; 02.11.2017 US 201762580574 P; 11.12.2017 WO PCT/EP2017/082148
(43) Date of publication of application: 19.02.2020
(73) Proprietor: Société des Produits Nestlé S.A., 1800 Vevey (CH)
(72) Inventor: RIEKER, Claus, 1025 St-Sulpice (CH); HUDRY-LABBE, Julie Laure, 1066 Epalinges (CH); SCHMITT, Jeroen Antonius Johannes, 1510 Moudon (CH); BOSCHAT, Corina, 1000 Lausanne (CH)
(74) Representative: Gordon, Kirsteen Helen
(86) International application number: PCT/EP2018/058710
(87) International publication number: WO 2018/189022

(56) References cited:
- WO-A1-2010/143053
- WO-A1-2016/207794
- US-A1- 2002 182 196
- US-A1- 2014 271 844
- US-A1- 2015 086 625
- KENNEDY: "B vitamins and brain", NUTRIENTS, 2016

## Description

### BACKGROUND

The present disclosure generally relates to compositions for use in attenuating cognitive aging in individuals who do not have dementia. More specifically, the present disclosure relates to attenuating cognitive aging by administering a composition comprising a combination of an omega-3 fatty acid, a nitric oxide releasing compound, an amount of Vitamin B12, choline, Vitamin B6 and Vitamin B9.

Population aging has been a remarkable demographic event. As the growth of the older population has outpaced the total population due to increased longevity, the proportion of older persons relative to the rest of the population has increased considerably due to decreased fertility rates. For example, one in every twelve individuals was at least 60 years of age in 1950, and one in every ten was aged 60 years or older by the end of 2000. By the end of 2050, the number of persons worldwide that is 60 years or over is projected to be one in every five.

Aged or aging individuals frequently suffer some degree of cognitive impairment, including decline in cognitive function that progresses with age; and age-related changes in brain morphology and cerebrovascular function are commonly observed. Cognitive decline has been consistently reported with aging across a range of cognitive domains including processing speed, attention, episodic memory, spatial ability and executive function. Brain imaging studies have revealed that these normal age-related cognitive declines are associated with decreases in both grey and white matter volume in the brain, with the fronto-striatal system most heavily compromised with aging. These decreases in cortical volume can be attributed to a number of detrimental cellular processes involved with normal aging, such as accumulation of damage by free radicals over time leading to oxidative damage, chronic low-grade inflammation, homocysteine accumulation (which when elevated are a risk factor for cognitive impairment and dementia), and decreased mitochondrial efficiency. In addition to direct cellular damage, the brain is also indirectly impaired by insults to micro-vascular structures. It is evident that the pathology of aging and also dementia involves a complexity of these interacting factors, which are linked together. For example, mitochondrial dysfunction leads to increased oxidative stress, and oxidative stress can trigger inflammation and vascular insults.

Furthermore, cognitive decline is an early predictor for Alzheimer pathology and begins before the onset of dementia. In this context, the cognitive composite score represents a reliable means to assess the cognitive decline preceding dementia. Considerable evidence suggests that maintaining brain health and preventing cognitive decline with advancing age may prevent or delay development of dementia due to Alzheimer's disease and other aged related neuropathologies.

Nutrition, education, physical exercise and cognitive exercise have been recently demonstrated as possible intervention to prevent cognitive decline with aging. An abundance of clinical, epidemiological, and individual evidence is in favor of individual nutritional factors that reduce dementia risk and age-related neurodegeneration. However, formal trial testing of nutritional interventions has yielded mixed results (Schmitt et al., Nutrition Reviews 68: S2-S5 (2010).

Several long-term studies have failed to observe any cognitive benefits with interventions using combinations of B6, B12 and folate. McMahon et al. (2006) N Engl J Med, 354(26), 2764-2772, found no effect on cognition in adults aged 65+ after 2 years consumption of a supplement containing folate (1000µg), Vitamin B12 (500µg) and B6 (10mg). Similarly, Hankey et al. (2013) (Stroke, 44(8), 2232-2239) found that daily supplementation with folic acid (2000µg), Vitamin B6 (25mg), and Vitamin B12 (500µg), to cognitively unimpaired patients with previous stroke or transient ischemic attack, lowered mean tHcy but had no effect on the incidence of cognitive impairment or cognitive decline, as measured by the MMSE, during a median of 2.8 years.

Several short-term studies have also failed to show an effect of the combination of B6, B12 and folate for improving cognitive function. Lewerin et al. (2005) Am J Clin Nutr, 81(5), 1155-1162, found that 4 months of supplementation of folic acid (800µg), Vitamin B12 (500µg), and Vitamin B6 (3mg) had no effect on cognition in older adults (median age 76 years).

US 2015/086625 A1 and US 2014/271844 A1 relate to methods for using activated fatty acids to treat a variety of diseases.

WO 2010/143053 A1 relates to compositions comprising (i) an activator of transport of fatty acids into the mitochondria; (ii) an activator of fatty acid oxidation; and (iii) an inhibitor of fatty acid biosynthesis, and methods for treating insulin resistance, diabetes mellitus type 2, metabolic syndrome, dyslipidemias, obesity, and/or cardiovascular disorders in a subject.

US 2002/182196 A1 relates to a nutritional supplement composition for normalizing impaired or deteriorating neurological function in humans, composed of: at least one agent which promotes synthesis of ATP and/or creatine phosphate in the body, at least one antioxidant for scavenging free radicals in at least one pathway in the body; at least one agent for normalizing or maintaining membrane function and structure in the body; at least one agent for normalizing or maintaining normal neurotransmitter function in the body; at least one agent for down-regulating cortisol action; and at least one agent for suppressing activation of apoptotic pathways in the body.

WO 2016/207794 A1 relates to a method for enhancing neurogenesis in an animal comprising identifying an animal that is suffering from cognitive decline, that is at risk of becoming cognitively impaired, that is diagnosed with a disease, disorder, or other condition that affects its cognitive abilities, or that is at risk of contracting a disease, disorder, or other condition that affects its cognitive abilities, and administering a composition comprising an unsaturated fatty acid (UFA), a nitric oxide releasing compound (NORC), a B vitamin, and an antioxidant, to the animal, wherein the composition is administered in an amount effective for enhancing neurogenesis in the animal.

### SUMMARY

Without being bound by theory, the present inventors believe that prior nutritional interventions attempting to reduce dementia risk and age-related neurodegeneration have focused on the administration of nutrients in isolation rather than together intelligently in combination to catapult the magnitude of effect by nutrient interaction. Moreover, studies investigating the effects of combined ingredients on cognitive function have used a mixture of constituents that all target the same mechanism (e.g. a mix of folate, B12, B6 mix targeting Hcy levels, or a mix of Vitamins C and E targeting oxidative damage), which may be why this evidence is as inconsistent as the single ingredient research. Therefore, the present disclosure is generally directed to a multi-intervention approach whereby each of the nutritional interventions targets a different risk factor associated with cognitive decline.

Accordingly, in a general embodiment, the present disclosure provides a composition for use in attenuating, treating or preventing cognitive aging in a non-demented individual in need thereof or at risk thereof comprising an omega-3 fatty acid; a nitric oxide releasing compound selected from the group comprising at least one or more of citrulline, arginine or ornithine; Vitamin B12 and choline, wherein the composition is administered in a daily dose that provides about 0.02 to 0.07 mg of Vitamin B12 per day, more preferably 0.03 to 0.05 mg of Vitamin B12 per day and 5.5 mg/day to 5,500 mg/day of the choline, preferably 85 mg/day to 3,500 mg/day of choline and additional Vitamin B6 and Vitamin B9. Subject to the aforementioned requirements, the composition is preferably administered in a daily dose that provides 0.1 to 50 times the recommended daily requirement (RDA) of Vitamin B12 per day, more preferably 0.1 to 40 times the recommended daily requirement (RDA) of Vitamin B12 per day and 0.01 to 10.0 times the recommended daily requirement (RDA) of choline.

In a more preferred embodiment, the daily dose provides about 10, 20, 30 or 40 times the RDA of Vitamin B12 per day, preferably 10 to 40, more preferably 10 to 30 or even more preferably 10 to 25 times the RDA of Vitamin B12 per day, most preferably about 12 to 21 times the RDA of Vitamin B12.

In another preferred embodiment the choline can be provided by an ingredient selected from the group consisting of choline chloride, choline bitartrate, citicoline (CDP-choline), L-alpha-glycerophosphocholine (Alpha-GPC), lecithin, phosphatidylcholine, and mixtures thereof, preferably choline bitartrate. Moreover, the composition is administered to the individual in a daily dose that provides 5.5 mg/day to 5,500 mg/day of the choline, preferably 85 mg/day to 3,500 mg/day of choline. Subject to the aforementioned requirements, and as defined before, the composition may be administered to the individual in a daily dose that provides 0.01 to 10.0 times the recommended daily requirement (RDA) of choline, for example 0.15 to 6.0 times the RDA of choline. In this regard, the RDA of choline is 550 mg/day.

In an embodiment, the individual is an older adult, for example an elderly human.

In an embodiment, the individual has a low DHA status at baseline. In an embodiment, the individual has a Clinical Dementia Rating (CDR) of 0.5 at baseline. In an embodiment the individual has a plasma homocysteine level at baseline of at least 12 µmol/L. In an embodiment, the individual has a risk score in Cardiovascular Risk Factors, Aging and Dementia (CAIDE) of 10 to 15 at baseline. In an embodiment, the individual is amyloid positive on amyloid PET scans at baseline. In an embodiment, the individual has a genotype indicating risk of cognitive decline.

In an embodiment, the composition is orally administered to the individual daily for at least one month.

In an embodiment, the nitric oxide releasing compound comprises citrulline.

In an embodiment, the omega-3 fatty acid comprises a fatty acid selected from the group consisting of docosahexaenoic acid, eicosapentaenoic acid and mixtures thereof.

In an embodiment, the composition comprises one or more additional B vitamins selected from the group consisting of Vitamin B1, Vitamin B2, Vitamin B3, Vitamin B5, and Vitamin B7; preferably all of Vitamin B1, Vitamin B2, Vitamin B3, Vitamin B5, Vitamin B6, Vitamin B7 and Vitamin B9.

In an embodiment, the composition comprises in addition to choline one or more further antioxidants selected from the group consisting of Vitamin C, Vitamin D, Vitamin E, and selenium.

A method of attenuating, treating or preventing cognitive aging in a non-demented individual in need thereof or at risk thereof, can comprise administering to the individual a therapeutically effective amount of a composition as hereinbefore defined.

An advantage of one or more embodiments provided by the present disclosure is to attenuate cognitive aging in non-demented individuals such as an elderly human.

Another advantage of one or more embodiments provided by the present disclosure is to use omega-3 fatty acids such as docosahexaenoic acid (DHA) and eicosapentaenoic acid (EPA) to modulate neuronal membrane fluidity, stimulate neuroplasticity, provide anti-neuroinflammatory effects, and/or reduce brain oxidative stress, in combination with B vitamins such as B12 to decrease homocysteine levels in the plasma and with a nitric-oxide releasing compound such as arginine or citrulline to protect signal transduction pathways.

Yet another advantage of one or more embodiments provided by the present disclosure is to use an amount of Vitamin B12 relative to known nutritional interventions for cognitive aging together with choline and omega-3 fatty acids such as docosahexaenoic acid (DHA) and eicosapentaenoic acid (EPA).

Still another advantage of one or more embodiments provided by the present disclosure is to decrease brain atrophy and neuroinflammation and increase or maintain amyloid-β phagocytosis and the number of synapses in a non-demented individual. In this context, one or more of the benefits achieved hereby are selected from the group consisting of improvement of neuronal fluidity, stimulation of neuronal plasticity and activity, improvement of the anti-inflammatory potential, reduction of reactive oxygen species (ROS) (e.g. by addition of choline), and/or target NO release or from other benefits as described herein.

Additional features and advantages are described herein and will be apparent from the following Figures and Detailed Description.

### BRIEF DESCRIPTION OF THE FIGURES

Figure 1 depicts the quantification of neuronal functionality for human neurons treated with different compounds. On the x axis are the time points when the measurement was performed, and on the y axis is the square root of number of spikes.
   T9 treatment shows significantly higher neuronal activity on time point 1, 4 and 24 hours compared to other treatments and control. * = p<0.05.
Figure 2 depicts the quantification of the data for human astrocytes treated for 24hrs with respective compounds as described above. On the x axis are the individual treatments, and on the y axis is the chemiluminescence. On the left side, there is the effect of the treatment upon the pro-inflammatory molecules IFNy and VCAM-1.
   T9 treatment lowers significantly the amount of IFNγ and VCAM-1 after treatment of 24 hours. *** = p<0.001.

### DETAILED DESCRIPTION

### Definitions

Some definitions are provided hereafter. Nevertheless, definitions may be located in the "Embodiments" section below, and the above header "Definitions" does not mean that such disclosures in the "Embodiments" section are not definitions.

All percentages expressed herein are by weight of the total weight of the composition unless expressed otherwise. As used herein, "about," "approximately" and "substantially" are understood to refer to numbers in a range of numerals, for example the range of -10% to +10% of the referenced number, preferably -5% to +5% of the referenced number, more preferably -1% to +1% of the referenced number, most preferably -0.1% to +0.1% of the referenced number. All numerical ranges herein should be understood to include all integers, whole or fractions, within the range. Moreover, these numerical ranges should be construed as providing support for a claim directed to any number or subset of numbers in that range. For example, a disclosure of from 1 to 10 should be construed as supporting a range of from 1 to 8, from 3 to 7, from 1 to 9, from 3.6 to 4.6, from 3.5 to 9.9, and so forth.

As used in this disclosure and the appended claims, the singular forms "a," "an" and "the" include plural referents unless the context clearly dictates otherwise. Thus, for example, reference to "a component" or "the component" includes two or more components.

The words "comprise," "comprises" and "comprising" are to be interpreted inclusively rather than exclusively. Likewise, the terms "include," "including" and "or" should all be construed to be inclusive, unless such a construction is clearly prohibited from the context. Nevertheless, the compositions disclosed herein may lack any element that is not specifically disclosed herein. Thus, a disclosure of an embodiment using the term "comprising" includes a disclosure of embodiments "consisting essentially of" and "consisting of" the components identified. A composition "consisting essentially of" contains at least 50 wt.% of the referenced components, preferably at least 75 wt.% of the referenced components, more preferably at least 85 wt.% of the referenced components, most preferably at least 95 wt.% of the referenced components.

The term "and/or" used in the context of "X and/or Y" should be interpreted as "X," or "Y," or "X and Y". Where used herein, the terms "example" and "such as", particularly when followed by a listing of terms, are merely exemplary and illustrative and should not be deemed to be exclusive or comprehensive.

The terms "food," "food product" and "food composition" mean a product or composition that is intended for ingestion by an individual such as a human and provides at least one nutrient to the individual. The compositions of the present disclosure, including the many embodiments described herein, can comprise, consist of, or consist essentially of the elements disclosed herein, as well as any additional or optional ingredients, components, or elements described herein or otherwise useful in a diet.

"Prevention" includes reduction of risk and/or severity of a condition or disorder. The terms "treatment," "treat," "attenuate" and "alleviate" include both prophylactic or preventive treatment (that prevent and/or slow the development of a targeted pathologic condition or disorder) and curative, therapeutic or disease-modifying treatment, including therapeutic measures that cure, slow down, lessen symptoms of, and/or halt progression of a diagnosed pathologic condition or disorder, and include treatment of patients at risk of contracting a disease or suspected to have contracted a disease, as well as patients who are ill or have been diagnosed as suffering from a disease or medical condition. The term does not necessarily imply that a subject is treated until total recovery. These terms also refer to the maintenance and/or promotion of health in an individual not suffering from a disease but who may be susceptible to the development of an unhealthy condition. These terms are also intended to include the potentiation or otherwise enhancement of one or more primary prophylactic or therapeutic measure. The terms "treatment," "treat," "attenuate" and "alleviate" are further intended to include the dietary management of a disease or condition or the dietary management for prophylaxis or prevention a disease or condition. A treatment can be patient- or doctor-related.

The term "individual" means any animal, including humans, that could suffer from cognitive aging and thus benefit from one or more of the methods disclosed herein. Generally, the individual is a human or an avian, bovine, canine, equine, feline, hicrine, lupine, murine, ovine or porcine animal. A "companion animal" is any domesticated animal, and includes, without limitation, cats, dogs, rabbits, guinea pigs, ferrets, hamsters, mice, gerbils, horses, cows, goats, sheep, donkeys, pigs, and the like. Preferably, the individual is a human or a companion animal such as a dog or cat, most preferably a human.

The term "elderly" in the context of a human means an age from birth of at least 60 years, preferably above 63 years, more preferably above 65 years, and most preferably above 70 years. The term "older adult" in the context of a human means an age from birth of at least 45 years, preferably above 50 years, more preferably above 55 years, and includes elderly individuals.

For other animals, an "older adult" has exceeded 50% of the average lifespan for its particular species and/or breed within a species. An animal is considered "elderly" if it has surpassed 66% of the average expected lifespan, preferably if it has surpassed the 75% of the average expected lifespan, more preferably if it has surpassed 80% of the average expected lifespan. An elderly cat or dog has an age from birth of at least about 7 years.

"Cognitive aging" is a decline in cognitive ability that progresses with age, for example an elderly age that is increasing, and can include age-related changes in brain morphology and/or cerebrovascular function. Cognitive aging does not include impaired cognitive ability caused by an underlying condition other than aging, such as a head injury or depression.

"Cognitive ability" is defined as the intellectual process by which an individual becomes aware of, perceives, or comprehends ideas. Cognitive ability embraces the quality of knowing, which includes all aspects of perception, recognition, conception, sensing, thinking, reasoning, remembering and imaging. Loss of cognitive ability is the difficulty in dealing with or reacting to new information or situations. Cognitive impairment may manifest itself in many ways, e.g., short-term memory loss, diminished capacity to learn, diminished rate of learning, diminished attention, diminished motor performance, and/or dementia, among other indicia. Non-limiting examples of specific cognitive domains that include abilities that decrease with age are (i) attention: processing speed, and selected and divided attention; (ii) learning and memory: delayed free recall, source memory, prospective memory, and episodic memory; (iii) language: verbal fluency, visitation naming, and word finding; (iv) visuospatial abilities: visual construction skills; and (v) executive functioning: planning, decision making, reasoning, and mental flexibility.

As used herein, an "effective amount" is an amount that prevents a deficiency, treats a disease or medical condition in an individual or, more generally, reduces symptoms, manages progression of the diseases or provides a nutritional, physiological, or medical benefit to the individual. The relative terms "improved," "increased," "enhanced" and the like refer to the effects of the composition disclosed herein relative to a composition lacking one or more ingredients and/or having a different amount of one or more ingredients, but otherwise identical.

### Embodiments

In the following paragraphs, when methods of treatment of the human or animal body are described as part of the invention, compounds or compositions for use in such methods are meant.

In an aspect of the present disclosure, a composition for use in attenuating, treating or preventing cognitive aging in a non-demented individual in need thereof or at risk thereof comprises an omega-3 fatty acid; a nitric oxide releasing compound selected from the group comprising at least one or more of citrulline, arginine or ornithine; Vitamin B12 and choline, wherein the composition is administered in a daily dose that provides about 0.02 to 0.07 mg of Vitamin B12 per day, more preferably 0.03 to 0.05 mg of Vitamin B12 per day and 5.5 mg/day to 5,500 mg/day of the choline, preferably 85 mg/day to 3,500 mg/day of choline and additional Vitamin B6 and and/or Vitamin B9.

The composition can increase cognitive function in a non-demented individual susceptible to or suffering from a decline in cognitive function brought about by the aging process. The composition can prevent, reduce or delay a decline in cognitive function in a non-demented individual susceptible to or suffering from a decline in cognitive function brought about by the aging process. In some embodiments, prior to the administration, individuals can be identified as having cognitive aging or being at risk of the cognitive aging. For example, prior to the administration, individuals can be identified as being in need of improved cognitive ability. The composition can decrease brain atrophy and neuroinflammation and increase amyloid-β phagocytosis and the number of synapses.

A method of treating cognitive aging in a non-demented individual in need thereof (e.g., having cognitive aging), can comprise administering to the individual a therapeutically effective amount of a composition as defined herein. As another example, a method of preventing cognitive aging in a non-demented individual at risk thereof (e.g., an older adult or an elderly individual but not yet having cognitive aging), can comprise administering to the individual a therapeutically effective amount of a composition as defined herein. As yet another example, a method of improving cognitive ability in a non-demented individual (e.g., an individual in need thereof) can comprise comprising administering to the individual a therapeutically effective amount of a composition as defined herein.

A "non-demented" individual has a Clinical Dementia Rating of up to 0.5. The CDR measures dementia severity and is a global rating of dementia with scores ranging from 0 to 3 (0, 0.5, 1, 2, and 3) rated by a semi-structured subject and informant interview. Hughes et al., Br. J. Psychiatry 140:566-72 (1982). A clinician synthesizes the cognitive and functional abilities based on six domains, including memory, orientation, judgment and problem solving, community affairs, home and hobbies, and personal care. The scale has good inter-rater agreement.

The non-demented individual does not have any of Alzheimer's disease, vascular dementia, Lewy body dementia, or frontotemporal dementia. In some embodiments, the non-demented individual is a healthy aging individual. In other embodiments, the non-demented individual has a phenotype associated with age-related cognitive impairment. For example, when compared to a control individual not having the phenotype, the non-demented individual may have a phenotype that includes one or more of decreased ability to recall, short-term memory loss, decreased learning rate, decreased capacity for learning, decreased problem solving skills, decreased attention span, decreased motor performance, or increased confusion.

A non-limiting example of a non-demented individual at risk of cognitive aging is a human with spontaneous memory complaints but who nevertheless has a Mini Mental State Examination (MMSE) score of at least 24 and has independence in basic daily activities as shown by an Activities of Daily Living (ADL) score of at least 4. An MMSE score for the present purpose may be e.g. 24 to 30, more preferably 26 to 30.

The MMSE is a very brief, easily administered/executed mental status examination that has proved to be a highly reliable and valid instrument for detecting and tracking the progression of the cognitive impairment associated with neurodegenerative diseases. The MMSE is a fully structured scale that consists of 30 points grouped into seven categories: orientation to place (state, county, town, hospital, and floor), orientation to time (year, season, month, day, and date), registration (immediately repeating three words), attention and concentration (serially subtracting 7, beginning with 100, or, alternatively, spelling the word world backward), recall (recalling the previously repeated three words), language (naming two items, repeating a phrase, reading aloud and understanding a sentence, writing a sentence, and following a three-step command), and visual construction (copying a design). Folstein et al., J. Psychiat. Res. 12:189-198 (1975).

The MMSE is scored in terms of the number of correctly completed items; lower scores indicate poorer performance and greater cognitive impairment. The total score ranges from 0 to 30.

The ADL is an informant-based activity of daily living scale widely used measure to assess activities of daily living in people with and without AD. The instrument assesses ability over a wide range of performances. The ADL has shown sensitivity to change among mildly impaired individuals compared to non-impaired controls and can capture functional changes. Galasko et al., Alzheimer Dis. Assoc. Disord. 11 Suppl. 2:S33-9 (1997).

As noted earlier herein, considerable evidence suggests that maintaining brain health and preventing cognitive decline with advancing age may prevent or delay development of dementia. Therefore, the compositions for use disclosed herein which treat or prevent cognitive aging can also ultimately prevent dementia such as Alzheimer's disease. Accordingly, a method of preventing dementia in an individual at risk thereof can comprise administering to the individual a therapeutically effective amount of the composition disclosed herein. The dementia that is prevented can be selected from the group consisting of Alzheimer's disease, vascular dementia, Lewy body dementia, frontotemporal dementia, and combinations thereof. The methods preferably comprise administering compositions as described herein.

In an embodiment, the individual has a low DHA status (erythrocyte omega 3 index < 4.8%) at baseline. In an embodiment, the individual has a Clinical Dementia Rating (CDR) of 0.5 at baseline. In an embodiment, the individual has a high plasma homocysteine level at baseline. As used herein, a "high" plasma homocysteine level is plasma homocysteine of at least 12 µmol/L. In another embodiment, the individual has a CAIDE (Cardiovascular Risk Factors, Aging and Dementia) risk score of 10-15 at baseline. In yet another embodiment, the individual is amyloid positive on amyloid PET scans at baseline. In yet another embodiment, the individual has a genotype indicating risk of cognitive decline (e.g., Apolipoprotein E (APOE) genotype).

The composition is administered in a daily dose that provides about 0.02 to 0.07 mg of Vitamin B12 per day. Subject to this requirement, a daily dose of the composition can provide 0.1 to 50 times the recommended daily requirement (RDA) of Vitamin B12 per day, more preferably 0.1 to 40 times the recommended daily requirement (RDA) of Vitamin B12. Such dosages may preferably include a daily dose of about 10, 20, 30, or 40 times the RDA of the Vitamin B12 per day. Preferably, the daily dose provides 10 to 40, more preferably 10 to 30 or even more preferably 10 to 25 times the RDA of the Vitamin B12 per day, most preferably about 12 to 21 times the RDA of the Vitamin B12 per day. Further in this regard, the United States RDA of Vitamin B12 is 2.4 micrograms daily for humans of age 14 years and older, so such individuals may be administered a daily dose of the composition that provides 0.02 to 0.07 mg of Vitamin B12 per day, more preferably 0.03 to 0.05 mg of Vitamin B12 per day.

In addition, the composition is administered to the individual in a daily dose that provides 5.5 mg/day to 5,500 mg/day of the choline, preferably 85 mg/day to 3,500 mg/day of choline. Subject to this requirement, and as defined before, the composition may be administered to the individual in a daily dose that provides 0.01 to 10.0 times the recommended daily requirement (RDA) of choline, for example 0.15 to 6.0 times the RDA of choline. In this regard, the RDA of choline is 550 mg/day.

In various embodiments, the omega-3 fatty acid is 1 to 50 wt.% of the composition, preferably 1 to 30 wt.% of the composition, and most preferably 1 to 15 wt.% of the composition. Preferably the omega-3 fatty acid comprises at least one of eicosapentaenoic acid (EPA) or docosahexaenoic acid (DHA) and more preferably comprises both EPA and DHA, each of which has anti-inflammatory properties. A daily dose of the composition preferably provides 0.5 g to 1.0 g of DHA per day and/or 0.5 g to 1.0 g of EPA per day, more preferably 0.7 g to 1.0 g of DHA per day and/or 0.6 mg to 0.75 g of EPA per day, and most preferably about 770 mg of DHA per day and/or about 700 mg of EPA per day.

The omega-3 fatty acid may comprise a blend of one or more sources of omega-3 fatty acids, and each of the one or more sources of omega-3 fatty acids can be natural (e.g., fish oil) or synthetic (i.e., formed through a chemical process manipulated by a human, as opposed to those of natural origin). The term "fish oil" means a crude or purified fatty or oily extract rich in omega-3 fatty acids and obtained from a sea individual, preferably a cold-water fish such as, but not limited to, salmon, tuna, mackerel, herring, sea bass, striped bass, halibut, catfish, and sardines, as well as shark, shrimp, and clams, or any combination thereof.

A nitric oxide releasing compound is any compound or compounds that cause or can result in the release of nitric oxide in an individual. The nitric oxide releasing compound is selected from the group consisting of at least one or more of citrulline, arginine or ornithine. Other nitric oxide releasing compounds not presently claimed are:
a peptide or protein containing at least one of these amino acids, preferably arginine and/or citrulline, and even more preferably comprises citrulline, which provides beneficial effects on the cardiovascular system, specifically in terms of improving blood flow, endothelial function and blood pressure. In various embodiments, the nitric oxide releasing compound is 1 to 20 wt.% of the composition, preferably 1 to 15 wt.% of the composition, and more preferably 1 to 10 wt.% of the composition. In an embodiment, a daily dose of the composition provides from 0.5 g to 10.0 g of the nitric oxide releasing compound (e.g., citrulline) per day, preferably 1.0 g to 5.0 g per day, more preferably 2.0 g to 4.0 g per day, and most preferably about 3.0 g per day.

The B vitamins can further comprise other B vitamins additional to the Vitamin B12, Vitamin B6, and Vitamin B9, for example one or more of Vitamin B1 (thiamine), Vitamin B2 (riboflavin), Vitamin B3 (niacin), Vitamin B5 (pantothenic acid), and Vitamin B7 (biotin) or salts, conjugates or derivatives thereof that have B vitamin activity. The composition can comprise from 0.1 to 40 times the RDA of one or more of these additional B vitamins, preferably 1 to 20 times the RDA, and more preferably 1 to 10 times the RDA.

In a most preferred embodiment, the B vitamins further comprise all of Vitamin B1 (thiamine), Vitamin B2 (riboflavin), Vitamin B3 (niacin), Vitamin B5 (pantothenic acid), Vitamin B6 (pyridoxine), Vitamin B7 (biotin) and Vitamin B9 (Folic acid), additional to the Vitamin B12.

The composition can comprise from 0.1 to 40 times the RDA of the Vitamin B6 and/or the Vitamin B9, preferably 1 to 20 times the RDA, and more preferably 1 to 10 times the RDA. For example, a daily dose of the composition can provide 15 mg to 25 mg of the Vitamin B6 per day, preferably 15 mg to 20 mg of the Vitamin B6 per day, most preferably about 18 mg of the Vitamin B6 per day. A daily dose of the composition can provide 0.1 mg to 1.0 mg of the Vitamin B9 per day, preferably 0.3 mg to 0.8 mg of the Vitamin B9 per day, most preferably about 0.4 mg of the Vitamin B9 per day. Each of Vitamins B6, B9 and B12 has the ability to decrease homocysteine levels in the plasma.

In some embodiments, the composition can further comprise one or more antioxidants in addition to choline to protect against oxidative damage and inflammation-induced damage. Non-limiting examples of suitable antioxidants include Vitamin C, Vitamin D, Vitamin E, selenium, , and combinations thereof. The composition can comprise 0.0001 wt.% to 25 wt.% of the antioxidant, if present; preferably 0.0001 wt.% to about 15 wt.%; more preferably 0.001 wt.% to 5 wt.%; and most preferably 0.001 wt.% to 2 wt.%.

In some embodiments, the composition is a food composition for a human and/or a pet such as a companion individual. The food composition may comprise one or more additional substances such as a mineral, another vitamin, a salt, or a functional additive such as flavoring, a colorant, an emulsifier, or an antimicrobial compound or other preservative. Non-limiting examples of suitable minerals include calcium, phosphorous, potassium, sodium, iron, chloride, boron, copper, zinc, magnesium, manganese and iodine. Non-limiting examples of suitable additional vitamins include fat soluble vitamins as A, D, E and K.

In another embodiment, the composition is a pharmaceutical composition comprising one or more pharmaceutically acceptable carriers, diluents, or excipients. Generally, pharmaceutical compositions are prepared by admixing the omega-3 fatty acid, the nitric oxide releasing compound, the Vitamin B12 and choline, as defined herein, with one or more of an excipient, a buffer, a binder, a plasticizer, a colorant, a diluent, a compressing agent, a lubricant, a flavorant, or a moistening agent.

The composition can have an acute effect that can be seen in less than one month. Additionally or alternatively, the composition can have a long-term effect, and thus various embodiments comprise administration of the composition to the individual (e.g., orally) for a time period of at least one month; preferably at least two months, more preferably at least three, four, five or six months; most preferably for at least one year. During the time period, the composition can be administered to the individual at least one day per week; preferably at least two days per week, more preferably at least three, four, five or six days per week; most preferably seven days per week. The composition can be administered in a single dose per day or in multiple separate doses per day.

Any of the embodiments as defined herein, particularly ingredients of the described compositions may be combined with each other, if not otherwise described. This also applies with regard to the features and benefits of the methods and treatments defined herein employing such compositions.

### EXAMPLES

### Example 1

The following non-limiting example is illustrative of compositions for attenuating cognitive aging in a non-demented individual, in embodiments provided by the present disclosure.

| **Ingredient** | **Dose/Day** |
|---|---|
| DHA | 770 mg |
| EPA | 700 mg |
| Vitamin B1 (thiamin) | 50 mg |
| Vitamin B2 (riboflavin) | 15 mg |
| Vitamin B3 (niacin) | 25 mg |
| Vitamin B5 (pantothenic acid) | 23 mg |
| Vitamin B6 (pyridoxine) | 18 mg |
| Vitamin B7 (biotin) | 0.15 mg |
| Vitamin B9 (folic acid anhydrous) | 0.4 mg |
| Vitamin B12 (cobalamin) | about 0.03 to 0.05 mg |
| Vitamin C | 500 mg |
| Vitamin D | 0.015 mg |
| Vitamin E | 82.6 mg |
| Selenium | 0.08 mg |
| Citrulline | 3000 mg |
| Choline bitartrate | 85 mg |

### Example 2 - Maintenance or increase of neuronal functionality

The following non-limiting example is an experimental example supporting the use of compositions defined herein to maintain neuronal functionality of cultured human induced-pluripotent stem cell derived neurons.

### Neurons and Molecular Reagents

Human induced-pluripotent stem cell derived neurons and accompanying growth media were purchased from Cellular Dynamics International. To measure neuronal functionality we use the Axion BioSystems' Maestro multielectrode array (MEA) technology, a noninvasive, label-free platform that measures the electrical activity. Neurons were cultured on a 48-well MEA plate (Axion BioSystems M768-KAP-48) according to the manufacturer's protocol (Cellular Dynamics International). Neurons were plated at a density of 100'000-120'000 cells/ per well, which have been coated with PLO/Laminin according to the manufactures protocol and maintained in an incubator (37°C, 5% CO₂). 50% of the maintenance media was exchanged twice per week.

Two weeks after plating media was completely exchanged and neuronal activity was measured for 15min (baseline). Afterwards the medium was fortified with: DHA+EPA, (1.5µM each); B6+B9,(20µM and 10µM); B12, (0.005µM); citrulline, (15µM); choline, (30µM); T9 containing DHA+EPA+B6+B9+B12+citrulline+choline, (15µM, 15µM, 20µM, 10uM, 0.005µM, 15µM, 30µM) control treatment (media only). Post-application recording was performed 1, 2, 4 and 24 hours for 15min.

Data analysis is performed by using the Axis software (Axion BioSystems) according to standard procedures.

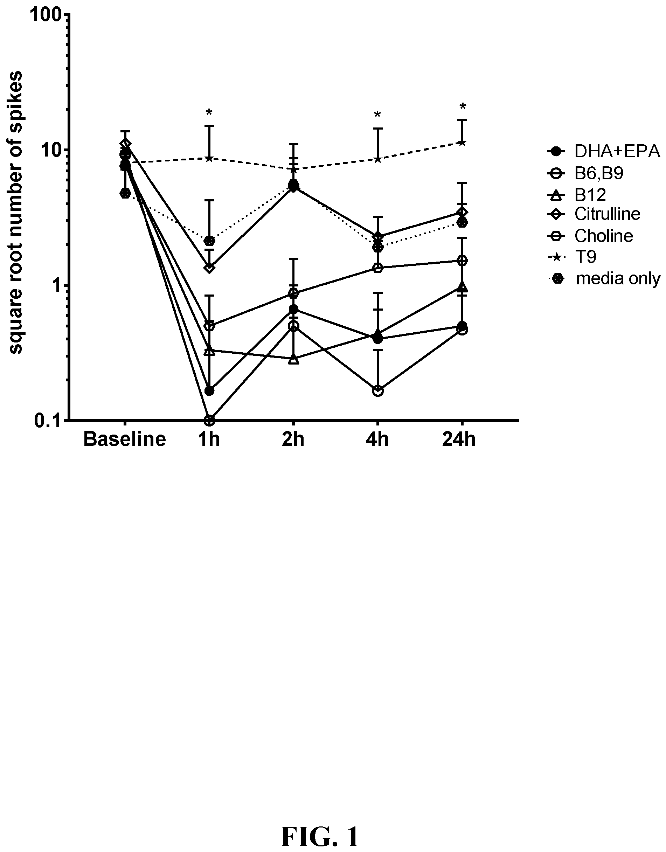

Data are presented as square root of spike counts ±SEM, N=6 in Figure 1. Statistical significance was computed using two-way ANOVA followed by Tukey's multiple-comparison testing. P less than 0.05 was considered significant.

As can be seen in Figure 1, T9 treatment shows significantly higher neuronal activity on time point 1, 4 and 24 hours compared to other treatments and control. * = p<0.05.

### Example 3 - Decrease of inflammatory signals

The following non-limiting example is an experimental example supporting the use of the inventive composition to lower inflammatory signals in cultured human induced-pluripotent stem cell derived astrocytes.

### Astrocytes and Molecular Reagents

Human induced-pluripotent stem cell derived astrocytes and accompanying growth media were purchased from Cellular Dynamics International. Astrocytes were cultured according to the manufacturer's protocol (Cellular Dynamics International). Astrocytes were plated at a density of 40'000-55'000 cells/cm² in culture vessels (6-well plastic wells, Greiner), which have been coated with PLO/Laminin according to the manufactures protocol and maintained in an incubator (37°C, 5% CO₂).

One week plating media was completely exchanged and replaced by media fortified with: DHA+EPA, (1.5µM each); B6+B9,(20µM and 10µM); B12, (0.005µM); citrulline, (15µM); choline, (30µM); T9 containing DHA+EPA+B6+B9+B12+citrulline+choline, (15µM, 15µM, 20µM, 10µM, 0.005µM, 15µM, 30µM) control treatment (media only).

After 24 hours cells were harvested and cell pellet processed using the Proteome Profiler Human XL Cytokine Array Kit (R&D systems; ARY022B) and chemiluminescence measured by Licor system.

Data are presented as bar graphs ±SEM. Statistical significance was computed using one-way ANOVA followed by Tukey's multiple-comparison testing. P less than 0.05 was considered significant.

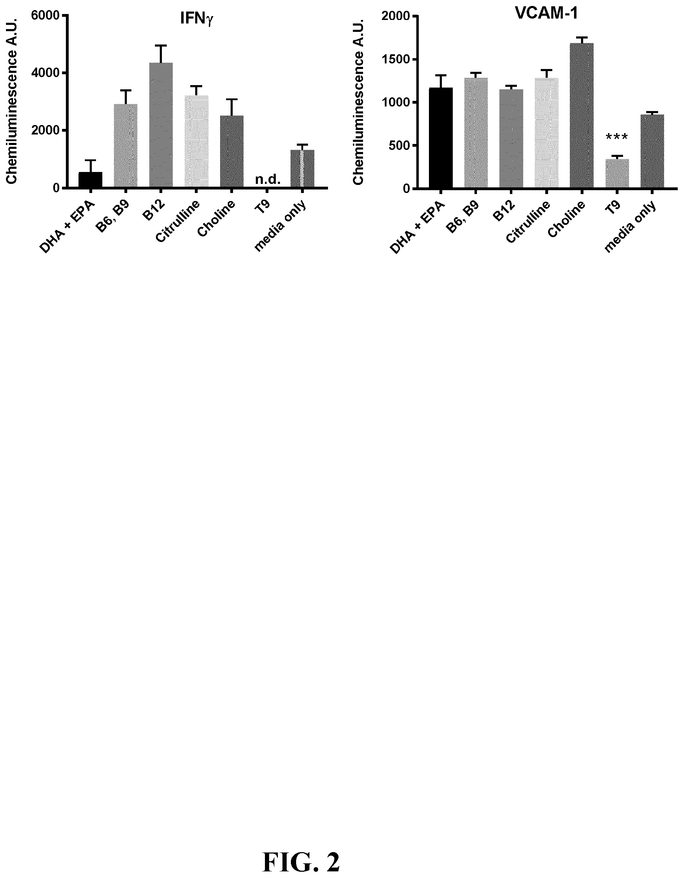

Figure 2 depicts the quantification of the data for human astrocytes treated for 24hrs with respective compounds as described above. On the X-axis are the individual treatments, and on the Y-axis is the chemiluminescence. On the left side, there is the effect of the treatment upon the pro-inflammatory molecules IFNy and VCAM-1.

T9 treatment lowers significantly the amount of IFNγ and VCAM-1 after treatment of 24 hours. *** = p<0.001. The results are shown in Figure 2.

## Claims

1. A composition for use in attenuating, treating or preventing cognitive aging in a non-demented individual in need thereof or at risk thereof comprising an omega-3 fatty acid; a nitric oxide releasing compound selected from the group consisting of at least one or more of citrulline, arginine or ornithine; Vitamin B12 and choline, wherein the composition is administered in a daily dose that provides about 0.02 to 0.07 mg of Vitamin B12 per day, more preferably 0.03 to 0.05 mg of Vitamin B12 per day and 5.5 mg/day to 5,500 mg/day of the choline, preferably 85 mg/day to 3,500 mg/day of choline and additional Vitamin B6 and Vitamin B9.

2. The composition for use according to Claim 1, wherein the daily dose provides about 0.03 to 0.05 mg of Vitamin B12 per day.

3. The composition for use according to Claim 1 or 2, wherein the daily dose provides about 85 to 3500 mg of choline per day.

4. The composition for use according to any of Claims 1 to 3, wherein the individual is an older adult or an elderly human.

5. The composition for use according to any of Claims 1 to 4, wherein the individual is a companion animal.

6. The composition for use according to any of Claims 1 to 5, wherein the composition is orally administered to the individual daily for at least one month.

7. The composition for use according to any of Claims 1 to 6, wherein the nitric oxide releasing compound comprises citrulline.

8. The composition for use according to any of Claims 1 to 7, wherein the omega-3 fatty acid comprises a fatty acid selected from the group consisting of docosahexaenoic acid, eicosapentaenoic acid and mixtures thereof.

9. The composition for use according to any of Claims 1 to 8, wherein the composition comprises one or more additional B vitamins selected from the group consisting of: Vitamin B1, Vitamin B2, Vitamin B3, Vitamin B5, and Vitamin B7.

10. The composition for use according to any of Claims 1 to 9, wherein the composition comprises one or more further antioxidants selected from the group consisting of Vitamin C, Vitamin D, Vitamin E, and selenium.

11. The composition for use according to any of Claims 1 to 10, wherein the individual has a low DHA status at baseline.

12. The composition for use according to any of Claims 1 to 11, wherein the individual has a Clinical Dementia Rating (CDR) of 0.5 at baseline.

13. The composition for use according to any of Claims 1 to 12, wherein the individual has a low a plasma homocysteine level at baseline of at least 12 µmol/L.

14. The composition for use according to any of Claims 1 to 13, wherein the individual has a risk score in Cardiovascular Risk Factors, Aging and Dementia (CAIDE) of 10 to 15 at baseline.

15. The composition for use according to any of Claims 1 to 14, wherein the individual is amyloid positive on amyloid PET scans at baseline.

16. The composition for use according to any of Claims 1 to 15, wherein the individual has a genotype indicating risk of cognitive decline.

17. The composition for use according to any of Claims 1 to 16, wherein the administration leads to an improvement of neuronal fluidity, stimulation of neuronal plasticity and activity, improvement of the anti-inflammatory potential, support or maintenance of cognitive performance, support or maintenance of brain performance, slowing down aging of the brain, support of an active mind and brain fitness, support or maintenance of a healthy brain, enhancement of memory, enhancement of executive functions, enhancement of attention, maintenance of cognitive health, maintenance of brain cellular health.

18. The composition for use according to Claim 17 for achieving one or more of benefits selected from the group consisting of: decreasing brain atrophy, increasing or maintaining number of synapses, increasing or maintaining amyloid-β phagocytosis, and decreasing neuroinflammation in a non-demented individual in need thereof, the method comprising administering to the individual a therapeutically effective amount of a composition comprising an omega-3 fatty acid; a nitric oxide releasing compound selected from the group consisting of:
citrulline, arginine or ornithine; wherein the composition is administered in a daily dose that provides about 0.02 to 0.07 mg of Vitamin B12 per day, more preferably 0.03 to 0.05 mg of Vitamin B12 per day and 5.5 mg/day to 5,500 mg/day of the choline, preferably 85 mg/day to 3,500 mg/day of choline and additional Vitamin B6 and Vitamin B9.

19. The composition for use according to Claim 18, wherein the composition is administered in a daily dose that provides about 0.03 to 0.05 mg of Vitamin B12 per day.

20. The composition for use according to Claim 18 or 19, wherein the composition is administered in a daily dose that provides about 85 mg/day to 3,500 mg/day choline.

21. The composition for use according to any of Claims 18 to 20, wherein the nitric oxide releasing compound comprises citrulline, and the omega-3 fatty acid comprises a fatty acid selected from the group consisting of docosahexaenoic acid, eicosapentaenoic acid and mixtures thereof.

22. The composition for use according to any one of claims 1 to 21, wherein the composition is a food product comprising an ingredient selected from the group consisting of protein, carbohydrate, fat and combinations thereof.

23. The composition for use according to any one of claims 1 to 21, wherein the composition is a pharmaceutical composition comprising a component selected from the group consisting of pharmaceutically acceptable carriers, diluents and excipients.

## Patentansprüche

1. Zusammensetzung zur Verwendung bei einem Abschwächen, Behandeln oder Vorbeugen kognitiver Alterung bei einem nicht-dementen Individuum, das dessen bedarf oder dem Risiko dessen ausgesetzt ist, umfassend eine Omega-3-Fettsäure; eine Stickstoffmonoxid freisetzende Verbindung, die aus der Gruppe ausgewählt ist, bestehend aus mindestens eines oder mehreren von Citrullin, Arginin oder Ornithin; Vitamin B12 und Cholin, wobei die Zusammensetzung in einer täglichen Dosis verabreicht wird, die etwa 0,02 bis 0,07 mg Vitamin B12 pro Tag, mehr bevorzugt 0,03 bis 0,05 mg Vitamin B12 pro Tag und 5,5 mg/Tag bis 5.500 mg/Tag des Cholins, vorzugsweise 85 mg/Tag bis 3.500 mg/Tag Cholin und zusätzliches Vitamin B6 und Vitamin B9 bereitstellt.

2. Zusammensetzung zur Verwendung nach Anspruch 1, wobei die tägliche Dosis etwa 0,03 bis 0,05 mg Vitamin B12 pro Tag bereitstellt.

3. Zusammensetzung zur Verwendung nach Anspruch 1 oder 2, wobei die tägliche Dosis etwa 85 bis 3500 mg Cholin pro Tag bereitstellt.

4. Zusammensetzung zur Verwendung nach einem der Ansprüche 1 bis 3, wobei das Individuum ein älterer Erwachsener oder ein älterer Mensch ist.

5. Zusammensetzung zur Verwendung nach einem der Ansprüche 1 bis 4, wobei das Individuum ein Begleittier ist.

6. Zusammensetzung zur Verwendung nach einem der Ansprüche 1 bis 5, wobei die Zusammensetzung an das Individuum täglich für mindestens einen Monat oral verabreicht wird.

7. Zusammensetzung zur Verwendung nach einem der Ansprüche 1 bis 6, wobei die Stickstoffmonoxid freisetzende Verbindung Citrullin umfasst.

8. Zusammensetzung zur Verwendung nach einem der Ansprüche 1 bis 7, wobei die Omega-3-Fettsäure eine Fettsäure umfasst, die aus der Gruppe ausgewählt ist, bestehend aus Docosahexaensäure, Eicosapentaensäure und Mischungen davon.

9. Zusammensetzung zur Verwendung nach einem der Ansprüche 1 bis 8, wobei die Zusammensetzung ein oder mehrere zusätzliche B-Vitamine umfasst, die aus der Gruppe ausgewählt sind, bestehend aus: Vitamin B1, Vitamin B2, Vitamin B3, Vitamin B5 und Vitamin B7.

10. Zusammensetzung zur Verwendung nach einem der Ansprüche 1 bis 9, wobei die Zusammensetzung ein oder mehrere weitere Antioxidantien umfasst, die aus der Gruppe ausgewählt sind, bestehend aus Vitamin C, Vitamin D, Vitamin E und Selen.

11. Zusammensetzung zur Verwendung nach einem der Ansprüche 1 bis 10, wobei das Individuum zu Behandlungsbeginn einen niedrigen DHA-Status aufweist.

12. Zusammensetzung zur Verwendung nach einem der Ansprüche 1 bis 11, wobei das Individuum zu Behandlungsbeginn eine klinische Bewertung der Demenz (CDR) von 0,5 aufweist.

13. Zusammensetzung zur Verwendung nach einem der Ansprüche 1 bis 12, wobei das Individuum zu Behandlungsbeginn einen niedrigen Plasma-Homocysteinspiegel von mindestens 12 µmol/L aufweist.

14. Zusammensetzung zur Verwendung nach einem der Ansprüche 1 bis 13, wobei das Individuum zu Behandlungsbeginn einen Risikowert bei kardiovaskulären Risikofaktoren, Alterung und Demenz (CAIDE) von 10 bis 15 aufweist.

15. Zusammensetzung zur Verwendung nach einem der Ansprüche 1 bis 14, wobei das Individuum zu Behandlungsbeginn bei Amyloid-PET-Scans Amyloidpositiv ist.

16. Zusammensetzung zur Verwendung nach einem der Ansprüche 1 bis 15, wobei das Individuum einen Genotyp aufweist, der ein Risiko für kognitiven Verfall angibt.

17. Zusammensetzung zur Verwendung nach einem der Ansprüche 1 bis 16, wobei die Verabreichung zu einer Verbesserung einer neuronalen Fluidität, einer Stimulation einer neuronalen Plastizität und Aktivität, einer Verbesserung des entzündungshemmenden Potenzials, einer Unterstützung oder Aufrechterhaltung einer kognitiven Leistungsfähigkeit, einer Unterstützung oder Aufrechterhaltung der Gehirnleistungsfähigkeit, einer Verlangsamung der Alterung des Gehirns, einer Unterstützung eines aktiven Geistes und einer Gehirnfitness, einer Unterstützung oder Aufrechterhaltung eines gesunden Gehirns, einer Verbesserung eines Gedächtnisses, einer Verbesserung exekutiver Funktionen, einer Verbesserung einer Aufmerksamkeit, einer Aufrechterhaltung einer kognitiven Gesundheit, einer Aufrechterhaltung einer zellulären Gesundheit des Gehirns führt.

18. Zusammensetzung zur Verwendung nach Anspruch 17 zum Erreichen eines oder mehrerer Vorteile, die aus der Gruppe ausgewählt sind, bestehend aus: einem Verringern einer Hirnatrophie, einem Erhöhen oder Aufrechterhalten einer Anzahl von Synapsen, dem Erhöhen oder Aufrechterhalten einer Amyloid-β Phagozytose und dem Verringern einer Neuroinflammation bei einem nicht-dementen Individuum, das dessen bedarf, das Verfahren umfassend das Verabreichen einer therapeutisch wirksamen Menge einer Zusammensetzung, umfassend eine Omega-3-Fettsäure, an das Individuum; eine Stickstoffmonoxid freisetzende Verbindung, die aus der Gruppe ausgewählt ist, bestehend aus:
Citrullin, Arginin oder Ornithin; wobei die Zusammensetzung in einer täglichen Dosis verabreicht wird, die etwa 0,02 bis 0,07 mg Vitamin B12 pro Tag, mehr bevorzugt 0,03 bis 0,05 mg Vitamin B12 pro Tag und 5,5 mg/Tag bis 5.500 mg/Tag des Cholins, vorzugsweise 85 mg/Tag bis 3.500 mg/Tag Cholin und zusätzliches Vitamin B6 und Vitamin B9 bereitstellt.

19. Zusammensetzung zur Verwendung nach Anspruch 18, wobei die Zusammensetzung in einer täglichen Dosis verabreicht wird, die etwa 0,03 bis 0,05 mg Vitamin B12 pro Tag bereitstellt.

20. Zusammensetzung zur Verwendung nach Anspruch 18 oder 19, wobei die Zusammensetzung in einer täglichen Dosis verabreicht wird, die etwa 85 mg/Tag bis 3.500 mg/Tag Cholin bereitstellt.

21. Zusammensetzung zur Verwendung nach einem der Ansprüche 18 bis 20, wobei die Stickstoffmonoxid freisetzende Verbindung Citrullin umfasst, und die Omega-3-Fettsäure eine Fettsäure umfasst, die aus der Gruppe ausgewählt ist, bestehend aus Docosahexaensäure, Eicosapentaensäure und Mischungen davon.

22. Zusammensetzung zur Verwendung nach einem der Ansprüche 1 bis 21, wobei die Zusammensetzung ein Nahrungsmittelprodukt ist, umfassend einen Bestandteil, der aus der Gruppe ausgewählt ist, bestehend aus Protein, Kohlenhydrat, Fett und Kombinationen davon.

23. Zusammensetzung zur Verwendung nach einem der Ansprüche 1 bis 21, wobei die Zusammensetzung eine pharmazeutische Zusammensetzung ist, umfassend eine Komponente, die aus der Gruppe ausgewählt ist, bestehend aus pharmazeutisch verträglichen Trägersubstanzen, Verdünnungsmitteln und Trägerstoffen.

## Revendications

1. Composition pour utilisation afin d'atténuer, traiter ou prévenir le vieillissement cognitif chez un individu non dément qui en a besoin ou qui est susceptible d'en avoir besoin, comprenant un acide gras oméga-3 ; un composé de libération d'oxyde nitrique choisi dans le groupe constitué d'au moins une ou plusieurs parmi la citrulline, l'arginine ou l'ornithine ; de la Vitamine B12 et de la choline, dans laquelle la composition est administrée dans une dose quotidienne qui fournit environ 0,02 à 0,07 mg de Vitamine B12 par jour, plus préférablement 0,03 à 0,05 mg de Vitamine B12 par jour et 5,5 mg/jour à 5500 mg/jour de choline, de préférence 85 mg/jour à 3500 mg/jour de choline et de Vitamine B6 et Vitamine B9 supplémentaires.

2. Composition pour utilisation selon la revendication 1, dans laquelle la dose quotidienne fournit environ 0,03 à 0,05 mg de Vitamine B12 par jour.

3. Composition pour utilisation selon la revendication 1 ou 2, dans laquelle la dose quotidienne fournit environ 85 à 3500 mg de choline par jour.

4. Composition pour utilisation selon l'une quelconque des revendications 1 à 3, dans laquelle l'individu est un adulte âgé ou une personne âgée.

5. Composition pour utilisation selon l'une quelconque des revendications 1 à 4, dans laquelle l'individu est un animal de compagnie.

6. Composition pour utilisation selon l'une quelconque des revendications 1 à 5, dans laquelle la composition est administrée par voie orale à l'individu quotidiennement pendant au moins un mois.

7. Composition pour utilisation selon l'une quelconque des revendications 1 à 6, dans laquelle l'oxyde nitrique libérant le composé comprend de la citrulline.

8. Composition pour utilisation selon l'une quelconque des revendications 1 à 7, dans laquelle l'acide gras oméga-3 comprend un acide gras choisi dans le **groupe constitué** d'acide docosahexaénoïque, acide eicosapentaénoïque et mélanges de ceux-ci.

9. Composition pour utilisation selon l'une quelconque des revendications 1 à 8, dans laquelle la composition comprend une ou plusieurs vitamines B supplémentaires choisies dans le groupe constitué de : Vitamine B1, Vitamine B2, Vitamine B3, Vitamine B5 et Vitamine B7.

10. Composition pour utilisation selon l'une quelconque des revendications 1 à 9, dans laquelle la composition comprend un ou plusieurs antioxydants supplémentaires choisis dans le groupe constitué de Vitamine C, Vitamine D, Vitamine E, et sélénium.

11. Composition pour utilisation selon l'une quelconque des revendications 1 à 10, dans laquelle l'individu a un faible statut en DHA au départ.

12. Composition pour utilisation selon l'une quelconque des revendications 1 à 11, dans laquelle l'individu a un indice de démence clinique (CDR) de 0,5 au départ.

13. Composition pour utilisation selon l'une quelconque des revendications 1 à 12, dans laquelle l'individu a un faible niveau d'homocystéine plasmatique au départ d'au moins 12 µmol/L.

14. Composition pour utilisation selon l'une quelconque des revendications 1 à 13, dans laquelle l'individu a un score de risque dans « Cardiovascular Risk Factors, Aging and Dementia (CAIDE) » (« Facteurs de risque cardiovasculaire, vieillissement et démence (CAIDE) ») de 10 à 15 au départ.

15. Composition pour utilisation selon l'une quelconque des revendications 1 à 14, dans laquelle l'individu est positif à l'amyloïde sur des balayages TEP à l'amyloïde au départ.

16. Composition pour utilisation selon l'une quelconque des revendications 1 à 15, dans laquelle l'individu a un génotype indiquant un risque de déclin cognitif.

17. Composition pour utilisation selon l'une quelconque des revendications 1 à 16, dans laquelle l'administration conduit à une amélioration de la fluidité neuronale, à une stimulation de la plasticité et de l'activité neuronales, à une amélioration du potentiel anti-inflammatoire, à un soutien ou à un maintien des performances cognitives, à un soutien ou à un maintien des performances cérébrales, à un ralentissement du vieillissement du cerveau, à un soutien d'un esprit actif et d'une bonne forme cérébrale, à un soutien ou à un maintien d'un cerveau sain, à un renforcement de la mémoire, à un renforcement des fonctions exécutives, à un renforcement de l'attention, à un maintien de la santé cognitive, à un maintien de la santé cellulaire cérébrale.

18. Composition pour utilisation selon la revendication 17, afin d'obtenir un ou plusieurs des bienfaits choisis dans le groupe constitué de : la diminution de l'atrophie cérébrale, l'augmentation ou le maintien du nombre de synapses, l'augmentation ou le maintien de la phagocytose de β amyloïde et la diminution de la neuroinflammation chez un individu non dément qui en a besoin, le procédé comprenant l'administration à l'individu d'une quantité thérapeutiquement efficace d'une composition comprenant un acide gras oméga-3 ; un composé de libération d'oxyde nitrique choisi dans le groupe constitué de :
citrulline, arginine ou ornithine ; dans laquelle la composition est administrée dans une dose quotidienne qui fournit environ 0,02 à 0,07 mg de Vitamine B12 par jour, plus préférablement 0,03 à 0,05 mg de Vitamine B12 par jour et 5,5 mg/jour à 5500 mg/jour de choline, de préférence 85 mg/jour à 3500 mg/jour de choline et des suppléments de Vitamine B6 et de Vitamine B9.

19. Composition pour utilisation selon la revendication 18, dans laquelle la composition est administrée dans une dose quotidienne qui fournit environ 0,03 à 0,05 mg de Vitamine B12 par jour.

20. Composition pour utilisation selon la revendication 18 ou 19, dans laquelle la composition est administrée dans une dose quotidienne qui fournit environ 85 mg/jour à 3500 mg/jour de choline.

21. Composition pour utilisation selon l'une quelconque des revendications 18 à 20, dans laquelle le composé de libération d'oxyde nitrique comprend de la citrulline, et l'acide gras oméga-3 comprend un acide gras choisi dans le groupe constitué d'acide docosahexaénoïque, acide eicosapentaénoïque et mélanges de ceux-ci.

22. Composition pour utilisation selon l'une quelconque des revendications 1 à 21, dans laquelle la composition est un produit alimentaire comprenant un ingrédient choisi dans le groupe constitué de protéine, glucide, matière grasse et combinaisons de ceux-ci.

23. Composition pour utilisation selon l'une quelconque des revendications 1 à 21, dans laquelle la composition est une composition pharmaceutique comprenant un composant choisi dans le groupe constitué de véhicules, diluants et excipients pharmaceutiquement acceptables.
